# EUROPEAN PATENT SPECIFICATION

(11) **EP 3 596 177 B1**
(45) Date of publication and mention of the grant of the patent: **29.11.2023**
(21) Application number: 17900819.8
(22) Date of filing: 16.03.2017
(51) Int. Cl.: C09D 133/10, C08L 71/02, C08F 220/28, C08F 265/06, C09D 4/00, C09D 4/06

(54) **ODORLESS UV CURABLE MONOMER SOLUTION**
GERUCHLOSE UV-HÄRTBARE MONOMERLÖSUNG
SOLUTION INODORE DE MONOMÈRE DURCISSABLE PAR UV

(43) Date of publication of application: 22.01.2020
(73) Proprietor: Mycone Dental Supply Company, Inc., Gibbstown, New Jersey 08027 (US)
(72) Inventor: SHERAN, Kevin, Haddonfield, New Jersey 08033 (US); GOUSE, Kendra, Philadelphia, Pennsylvania 19122 (US); CRESCIMANNO, Stephen, Hatfield, Pennsylvania 19440 (US)
(74) Representative: Boult Wade Tennant LLP
(86) International application number: PCT/US2017/022610
(87) International publication number: WO 2018/169530

(56) References cited:
- GB-A- 1 292 953
- US-A- 3 780 003
- US-A- 3 893 988
- US-A- 3 927 203
- US-A- 4 260 701
- US-A- 4 495 172
- US-A- 5 098 696
- US-A- 5 127 414
- US-A- 6 051 242
- US-B1- 6 455 033

## Description

### FIELD OF THE INVENTION

The present invention according to appended claims 1 to 14 is directed generally to the field of nail manicuring. More specifically, this invention applies to nail care products and processes for treating nails.

### DESCRIPTION OF RELATED TECHNOLOGY

Acrylic nails are used to artificially enhance the appearance of natural fingernails. The term "acrylic nail" covers a range of product types, including press-on nails, nail tips, and sculpted nails.

Press-on acrylic nails, introduced to the manicure industry in the early 1970s, are nail-shaped pieces of polymer that are glued on over natural nails. The subsequent development afforded more natural-looking nail enhancements which bond to the real nail by an acrylic-based resin. Such a resin is created by mixing a liquid and powder together to form a thick paste. A salon technician applies the paste over the natural nail and allows it to harden to form a durable nail coating finish that is filed into the desired shape.

Although press-on acrylic nails are used today, they have been largely replaced by sculpted acrylic nails. Sculpted acrylic nails may be formed by several different methods, but the most popular and widely-used method comprises an application of a two-part acrylic formulation by a nail technician. The nail technician typically wets a brush with a monomer liquid, dips the wetted brush into a polymeric powder, places the wetted ball of the mixture of monomer liquid and polymer powder onto a nail and an acrylic nail form adjacent to a nail, and shapes the artificial nail.

The acrylic nail form is a substrate used for construction acrylic nails beyond the existing free edge of the nail. Elongation requires affixing a substrate means to the exposed edge of the nail, which means has the general contour of the natural nail and extends therefrom to the desired length and along the plane of the natural nail. The substrate is typically of a material to which the polymerized mixture does not adhere, so that the form may be removed following construction of the artificial nail.

A traditional formulated monomer liquid used in a two-part acrylic system is mostly ethyl methacrylate ("EMA"). The monomer liquid typically also contains various other ingredients, such as additional monomers, crosslinkers, colorants, stabilizers, and polymerization accelerator or promoter such as a tertiary amine, to allow for proper performance in the hands of the nail technician and on the client's nails after application. The mixture of the monomer liquid and polymeric powder is typically cured thermally.

The powder portion of the two-part acrylic formulation generally comprises polymethyl methacrylate and a catalyst, such as benzoyl peroxide.

One of the biggest disadvantages of the monomer liquid used in the traditional system is the offensive odor of the ethyl methacrylate. This odor may be unappetizing to most clients and its long term exposure has been hypothesized to be dangerous to the nail technicians.

Although scientific studies and regulatory reviews by boards such as the Cosmetic Ingredient Review Board determined that ethyl methacrylate to be safe, consumers tend to eschew strong smelling substances either on aesthetic grounds, or due to perceived health risks.

Many attempts have been made to mitigate the odor exposure to the clients and nail technicians. For example, some monomer liquids have been formulated to include oil. Although oil may lower the odor somewhat, the use of oil also tends to have a negative impact on the formation of the artificial nail, especially with respect to the curing properties and surface properties, compared to the oil-free monomer liquids.

Another method of forming acrylic nails is by the use of a UV-curable system. In the UV-curable system method, a brush dipped into the formulated monomer liquid is placed into the formulated polymer powder, applied to the client's finger nail, and after the artificial nails are formed, the artificial nails are cured under a UV lamp for several minutes.

A UV-cured system has no odor compared to traditional acrylic systems. However, many nail technicians find that UV-curable systems are difficult to work with and are presently typically only used to create overlays, and not free standing nail extensions. Further, another disadvantage of typical UV-cured systems are brittleness of the formed nail.

Another approach taken by the formulators to solve the issue of odor was to use less volatile systems. In such two-part systems, the monomer liquid comprised other monomers instead of EMA. Examples of such systems include ethylene glycol dimethacrylate ("EGDMA"), and a mixture of 2-hydroxyethyl methacrylate ("HEMA") and 2-hydroxypropyl methacrylate ("HPMA"). Although such systems eliminated the EMA odor, allowing the systems to be marketed as "odorless acrylic systems", such systems have the disadvantages of a significant yellow color forming immediately upon application and with time on the nail. Another disadvantage of the odorless acrylic systems is that such systems tend to cure with a gummy/ rubbery surface that makes it difficult to file. This surface is more than likely due to oxygen inhibition during the curing of the product. The resultant nail extension is softer than the EMA-based product.

Other ingredients are added to the monomer liquid and the polymer powder to control the properties of the resin. Crosslinking agents are used to hook the polymer chains together to make the plastic more rigid. The most common is EGDMA. The polymer powder also carries an initiator, which starts the reaction that links the monomers together. A common initiator is benzoyl peroxide (BP), the same ingredient used in acne creams. When the liquid and powder are mixed together and applied to the client's fingers, the BP molecule is capable of exciting or energizing a monomer. Once energized, the monomers join together to form a polymer. Catalysts are also added to the formula to control the speed by which the initiator activates the reactions. A relatively small amount of catalyst is required to do the job, typically about only 1% of the monomer. Chemical inhibitors are added to the liquid monomer blend to prevent the monomers from reacting together prematurely, which turns the liquid into an unusable gel. Inhibitors help prolong the shelf life of the monomer solution. Plasticizers are used to improve resin performance. These liquids help lubricate the polymer chains so they are better able to resist breaking caused by stress.

Another technique to build acrylic nails involves the use of a dip acrylic system. To form nails according to this technique, the dip systems uses a many step procedure of layers of cyanoacrylate coatings alternating with layers of acrylic polymer.

Yet another technique to build acrylic nails involves the use of a gel powder system. According to this technique, the polymer powder is sprinkled on the applied monomer liquid rather than blending the monomer liquid with the polymer powder on a brush.

A formulation for self-curing artificial fingernails containing methoxyethyl methacrylate is taught in U.S. Patent Nos. 4,260,701. The disclosed composition for a fingernail coating has an acrylic binder, a peroxide catalyst, a tertiary amine accelerator, and a polymeric filler at least partially soluble in the coating. The acrylic binder contains a monoethylenically unsaturated monomer comprising at least a major proportion of methoxyethyl methacrylate. A polyfunctional monomer may be present that copolymerizes with the monoethylenically unsaturated monomer, for crosslinking and toughening.

Compositions and a process for applying protective covering and extensions to fingernails are disclosed in U.S. Patent No. 4,669,491. The disclosed process for applying a protective acrylic coating, with or without extending the tips, to the human fingernail as afforded by clearing and roughening the surface of the nail, applying a layer of liquid acrylic monomer, applying a powdered polymethacrylate ester to the wetted nail surface, removing loose powder and smoothing the nail surface, brushing on, as one would with nail polish, a second layer of liquid acrylic monomers and, after curing, finishing the nail in the conventional manner.

Odorless artificial fingernail composition and method of using the same are taught in U.S. Patent No. 4,871,534. The disclosed compositions are useful for making artificial fingernails, in particular such compositions that are odorless, non-toxic, self-curing, and demonstrate good working properties. Compositions consist of two parts: (a) an odorless, non-toxic liquid binder comprising one or more methacrylate monomer(s) of the following formula: R-O-CH₂-CH₂-O-CH₂CH2-O-CO-C(CH₃)=CH₂, where R is CH₃(CH₂)ₙ and n=0-3 together with one or more di-, tri-, or multi-functional methacrylates, and a tertiary-amine type accelerator; and (b) a polymeric powder containing a finely divided methacrylate polymer or copolymer, and a peroxide polymerization initiator.

A method of obtaining a tack-free artificial nail surface using odorless monomers is disclosed in U.S. Patent No. 7,622,511. A method and kit for providing a tack-free artificial nail surface is disclosed. The tack-free artificial nail surface may include an acrylic layer and a barrier layer. The acrylic layer may comprise an odorless monomer. The barrier layer may be of any material impervious to oxygen. The tack-free nail surface may be formed by application of the acrylic layer to a fingernail nail bed. The barrier layer may then be applied over the acrylic layer. The barrier layer blocks oxygen from the acrylic layer so that the acrylic layer may polymerize in the absence of oxygen.

Artificial nail compositions and related methods are disclosed in U.S. Patent No. 6,818,207. That patent discloses a polymerizable monomer composition for application to the nail surface and polymerization thereon to yield an artificial nail structure, comprising at least one multicarbonyl-vinyl containing monomer; a polymerized artificial nail structure having a thickness of about 10-60 mils, and a modulus of elasticity of about 550-800 N/m², comprising a copolymer of at least one ethylenically unsaturated monomer and a multicarbonyl vinyl-containing monomer; a method for reducing, ameliorating, or eliminating delamination of an artificial nail structure from the natural nail surface; a method for improving adhesion of an artificial nail structure to the nail surface; and a method for reducing premature gelation of a liquid monomer composition.

Synthesis and characterization of copolymers of methyl methacrylate and 2-ethoxyethyl methacrylate has been disclosed by M. Manju et al. in Am. J. Polymer Sci., 2012, vol. 2, iss. 3, pp 22 to 27. The free-radical initiated copolymerizations of methyl methacrylate with 2-ethoxyethyl methacrylate were carried out using 2,2-azobisisobutyronitrile as the initiator in 1,4-dioxane solutions. The reactivity ratios of the monomers were computed by the Fineman-Rose (F-R) and Kelen-Tudos (K-T) methods at lower conversion, using the IR data. The reactivity ratios indicate the formation of random copolymers, which have been supported by the azeotropic composition evaluations.

Synthesis, characterization, and evaluation of copolymers based on N-isopropylacrylamide and 2-ethoxyethyl methacrylate for the controlled release of felodipine has been taught by N. B. Shelke et al. in J. Appld. Polymer Sci., 2008, vol. 110, pp. 2211 to 2217. The free-radical copolymerization of N-isopropylacrylamide with 2-ethoxyethyl methacrylate was carried out with 2,2'-azobisisobutyronotrile as an initiator in 1,4-dioxane at 65° C. The resulting copolymer The distribution of the monomer sequence along the copolymer chain was calculated with a statistical method based on the obtained reactivity ratios. The effect of N-isopropylacrylamide with respect to 2-ethoxyethyl methacrylate segments on the sustained release of felodipine from the microspheres was investigated. The in vitro release studies of felodipine from the N-isopropylacrylamide/ 2-ethoxyethyl methacrylate microspheres performed in a pH 7.4 medium showed that the rate of drug release was enhanced by an decreasing the amount of 2-ethoxyethyl methacrylate. In US4495172A, US5127414A, US3893988A, US5098696A and US3780003A further monomer compositions for nail coatings are also described.

Although many advances in the art of formulating two-part acrylic nail system have been made to solve various problems, overcoming the odor while providing an acrylic nail with desired properties remain elusive.

### SUMMARY OF THE INVENTION

The present invention relates an acrylic nail monomer solution that can be used in a two-part acrylic system. The mixture of a monomer liquid of the present invention with a polymer enables a nail technician to form an artificial nail in a way that is similar to traditional two-part systems, but that cures under UV light.

Characteristics and/or advantages of the present invention may include significantly odors reduction associated with a traditional two-part acrylic nail formation; the improvement of cure properties; improvement of through-cure efficacy; an increase of surface hardness; a reduction of the yellowing; lower cost of the components; a simple application procedure; a long open time to sculpt the nail; a lower exotherm profile compared to similar nail formulations; and a curing of the mixture to form a hard acrylic nail that is easy to file.

In one aspect, the invention provides an acrylic nail monomer liquid comprising (a) about 50 wt% to about 65 wt% of 2-ethoxyethyl methacrylate, (b) about 17 wt% to about 35 wt% of hydroxypropylmethacrylate, (c) one or more crosslinkers, and (d) one or more curing agents, wherein all wt% are with respect to the acrylic nail monomer liquid and the term "about" when referring to a number means ±3%.

In one embodiment falling within the scope of the claims, the acrylic nail monomer liquid comprises (a) about 50 wt% to about 65 wt% of 2-ethoxyethyl methacrylate; (b) about 17 wt% to about 35 wt% of hydroxypropylmethacrylate; (c) about 0.15 wt% to about 0.30 wt% of triphenylphosphine; (d) a crosslinker; and (e) a photoinitiator; wherein all %wt are with respect to the acrylic nail monomer liquid.

2-Ethoxyethyl methacrylate ("ETMA") is a composition consisting of, or comprising largely of, the compound of formula CH₂=CMe-C(O)-O-(CH₂)₂-O-Et. ETMA, also abbreviated as 2-EOEMA, has both ether and ester groups as compared to most of the vinyl acrylic monomers. The amount of ETMA is about 50 wt% to about 65 wt% with respect to the monomer liquid. Under an alternative embodiment the amount of ETMA is about 55 wt% to about 60 wt%.

Hydroxypropyl methacrylate, or HPMA, is a composition consisting of, or comprising largely of, the compound of formula CH₂=CMe-C(O)-O-C₃H₆OH. The hydroxypropyl portion of the methacrylate, -C₃H₆OH, has the structure -CH₂-CH₂-CH₂-OH,
-CH₂-CH(OH)-CH₃ ,-CH(OH)-CH₂-CH₃ , -CH(CH₃)-CH₂-OH , -C(OH)(CH₃)-CH₃ , or a mixture of any of the foregoing. The amount of HPMA is about 17 wt% to about 35 wt% with respect to the monomer liquid. Under an alternative embodiment the amount of HPMA is about 21 wt% to about 30 wt%.

The monomer liquid of the present invention also comprises one or more crosslinkers. A crosslinker is a compound that contains two or more acrylate groups. Crosslinkers are necessary to provide chemical bonding between several monomers, oligomers, polymers or combinations thereof to yield a cross-linked polymeric structure. Examples of crosslinkers include diacrylates, triacrylates, tetraacrylates, pentaacrylates and higher acrylates. Such examples include trimethylolpropane triacrylate, trimethylolethane triacrylate, trimethylolpropane trimethacrylate, trimethylolethane trimethacrylate, tetramethylene glycol dimethacrylate, triethylene glycol dimethacrylate, tetraethylene glycol diacrylate, pentaerythritol diacrylate, penta-erythritol triacrylate, pentaerythritol tetraacrylate, dipentaerythritol diacrylate, dipenta-erythritol triacrylate, dipentaerythritol tetraacrylate, dipentaerythritol pentaacrylate, dipenta-erythritol hexaacrylate, tripentaerythritol octaacrylate, pentaerythritol dimethacrylate, penta-erythritol trimethacrylate, dipentaerythritol dimethacrylate, dipentaerythritol tetramethacrylate, tripentaerythritol octamethacrylate, ethylene glycol diacrylate, 1,3-butanediol diacrylate, 1,3-butanediol dimethacrylate, sorbitol triacrylate, sorbitol tetraacrylate, pentaerythritol-modified triacrylate, sorbitol tetramethacrylate, sorbitol pentaacrylate, sorbitol hexaacrylate, oligoester acrylates and methacrylates, glycerol di- and tri-acrylate, 1,4-cyclohexane diacrylate, bisacrylates and bismethacrylates of polyethylene glycol having a molecular weight of from 200 to 1500, and mixtures thereof.

The acrylic nail monomer of the present invention also comprises one or more curing agents. Examples of a suitable curing agent includes phosphorus-based curing agent, such as triphenylphosphine, tributylphosphine, tri(*p*-methylphenyl)phosphine, tri(nonylphenyl)phosphine, diphenyltolylphosphine, tetraphenylphosphonium bromide, methyltriphenylphosphonium, methyltriphenylphosphponium chloride, methoxymethyltriphenylphosphonium, and benzyltriphenylphosphonium chloride; or an amine-based curing agent, such as melamine, imidazole, N,N-dimethyl-p-toluidine (DMPT), N-methyl-N-(2-hydroxyethyl)-p-toluidine (MHPT), and isomeric toluidines.

The monomer liquid of the present invention may also comprise one or more photoinitiators. Such photoinitiators may be selected from benzyl ketones, monomeric hydroxyl ketones, polymeric hydroxyl ketones, α-amino ketones, acyl phosphine oxides, metallocenes, benzophenone, and benzophenone derivatives.

The monomer solution of the present application may further comprise additional ingredients, including colorants, dyes, pigments, whiteners, and perfumes. Dyes and pigments may be included to alter the appearance of the formed artificial nail. Whiteners may be added to the solution to whiten the nail and create a more natural appearance. Other colorants may be added to give the nail coating a pinkish color cast. Alternatively, blue or violet colorants, may be added to the monomer solution to counter any yellowing.

Yellowing of the artificial nail may also be mitigated by adding color stabilizers to the monomer liquid. Such compounds absorb ultraviolet light that can cause discoloration of the resins.

Other additives include flow agents to help control how the mixture of the monomer liquid and polymer powder spreads on the surface of the nail.

Another aspect of the present invention is a method of use of the above-described acrylic nail monomer liquid to form an acrylic nail coating. This method comprises mixing the acrylic nail monomer liquid with a polymeric powder to create a mixture; placing the mixture onto a nail; and exposing the mixture to a UV light.

Another aspect of the present invention is an acrylic nail coating formed by a method comprising the steps of mixing the acrylic nail monomer liquid with a polymeric powder to create a mixture; placing the mixture onto a nail; and exposing the mixture to a UV light to form the acrylic nail coating.

The present invention is also directed to the acrylic nail monomer liquid which when used with a polymer, exhibits a lower exotherm compared to similar products. The exotherm is the apparent rise in temperature due to the heat generated during the curing reaction caused by UV light.

The present invention is also directed to the acrylic nail monomer liquid which when used with a polymer resists the yellowing of the nail coating. Such mitigation of yellowing may be observed over time by the use of procedures such as those in ASTM E313.

In the first aspect, the invention relates to an acrylic nail monomer liquid comprising about 50 wt% to about 65 wt% of 2-ethoxyethyl methacrylate; about 17 wt% to about 35 wt% of hydroxypropylmethacrylate; one or more crosslinkers; and one or more curing agents; wherein all wt% are with respect to the acrylic nail monomer liquid.

In this aspect of the invention, and the below embodiments, the term "about" when referring to a number means ±3%.

In a further embodiment falling within the scope of the claims, the acrylic nail monomer liquid comprises about 55 wt% to about 60 wt% of 2-ethoxyethyl methacrylate; about 17 wt% to about 35 wt% of hydroxypropylmethacrylate; one or more crosslinkers; and one or more curing agents; wherein all wt% are with respect to the acrylic nail monomer liquid.

In a further embodiment falling within the scope of the claims, the acrylic nail monomer liquid comprises about 50 wt% to about 65 wt% of 2-ethoxyethyl methacrylate; about 21 wt% to about 30 wt% of hydroxypropylmethacrylate; one or more crosslinkers; and one or more curing agents; wherein all wt% are with respect to the acrylic nail monomer liquid.

In a further embodiment falling within the scope of the claims, the acrylic nail monomer liquid comprises about 50 wt% to about 65 wt% of 2-ethoxyethyl methacrylate; about 17 wt% to about 35 wt% of hydroxypropylmethacrylate; a polyacrylate selected from the group consisting of a diacrylate, triacrylate, tetraacrylate, pentaacrylate, and a mixture thereof; and one or more curing agents; wherein all wt% are with respect to the acrylic nail monomer liquid.

In a further embodiment falling within the scope of the claims, the acrylic nail monomer liquid comprises about 50 wt% to about 65 wt% of 2-ethoxyethyl methacrylate; about 17 wt% to about 35 wt% of hydroxypropylmethacrylate; one or more crosslinkers wherein one crosslinker is an alkoxylated crosslinker of formula (CH₂=CMe-C(O)-O-(AO)ₓ-CH₂-)₃C-R; wherein R is a C₁ to C₆ alkyl group; AO is an alkoxy group selected from the group consisting of ethylene oxide, -CH₂-CH₂-O-, propylene oxide, -CH(CH₃)-CH₂-O-, -CH₂-CH₂-CH₂-O-, butylene oxide, and -CH(Et)-CH₂-O-; wherein for each CH₂=CMe-C(O)-O-(AO)ₓ-CH₂- group x is independently 0, 1, 2, or 3; and one or more curing agents; wherein all wt% are with respect to the acrylic nail monomer liquid.

In a further embodiment falling within the scope of the claims, the acrylic nail monomer liquid comprises about 50 wt% to about 65 wt% of 2-ethoxyethyl methacrylate; about 17 wt% to about 35 wt% of hydroxypropylmethacrylate; one or more crosslinkers; where one crosslinker is ethoxylated crosslinker of formula (CH₂=CMe-C(O)-O-(CH₂-CH₂-O)*ₓ*-CH₂-)₃C-C₂H₅; wherein for each CH₂=CMe-C(O)-O-(CH₂-CH₂-O)*ₓ-*CH₂- group x is independently 0, 1, 2, or 3; and one or more curing agents; wherein all wt% are with respect to the acrylic nail monomer liquid.

In a further embodiment falling within the scope of the claims, the acrylic nail monomer liquid comprises about 50 wt% to about 65 wt% of 2-ethoxyethyl methacrylate; about 17 wt% to about 35 wt% of hydroxypropylmethacrylate; one or more crosslinkers; and one or more curing agents selected from the group consisting of triphenylphosphine, tributylphosphine, tri(p-methylphenyl)phosphine, tri(nonylphenyl)phosphine, diphenyltolylphosphine, tetraphenylphosphonium bromide, methyltriphenylphosphonium, methyltriphenylphosphponium chloride, methoxymethyltriphenylphosphonium, benzyltriphenylphosphonium chloride, melamine, imidazole, N,N-dimethyl-p-toluidine (DMPT), N-methyl-N-(2-hydroxyethyl)-p-toluidine (MHPT), isomeric toluidines, and mixtures thereof; wherein all wt% are with respect to the acrylic nail monomer liquid.

In a further embodiment falling within the scope of the claims, the acrylic nail monomer liquid comprises about 50 wt% to about 65 wt% of 2-ethoxyethyl methacrylate; about 17 wt% to about 35 wt% of hydroxypropylmethacrylate; one or more crosslinkers; and about 0.15 wt% to about 0.30 wt% of one or more curing agents, wherein the one or more curing agents is triphenylphosphine; wherein all wt% are with respect to the acrylic nail monomer liquid.

In a further embodiment falling within the scope of the claims, the acrylic nail monomer liquid comprises about 50 wt% to about 65 wt% of 2-ethoxyethyl methacrylate; about 17 wt% to about 35 wt% of hydroxypropylmethacrylate; one or more crosslinkers; one or more curing agents; and a photoinitiator, selected from the group consisting of 1-hydroxy-cyclohexylphenylketone,; benzophenone; 2-benzyl-2-(dimethylamino)-1-(4-(4-morphorlinyl)phenyl)-1-butanone; 2,2-dimethoxy-2-phenyl acetophenone; 2-methyl-1-(4-methylthio)phenyl-2-(4-morphorlinyl)- 1 -propanone; 2,4,6-trimethylbenzoyldiphenyl-phosphine oxide; bis(2,4,6-trimethyl benzoyl)phenyl phosphine oxide; diphenyl-(2,4,6-trimethylbenzoyl) phosphine oxide; bis(2,6-dimethoxybenzoyl-2,4,4-trimethyl pentyl)phosphine oxide; 2-hydroxy-2-methyl-1-phenyl-propan-1-one; phenyl bis(2,4,6-trimethylbenzoyl) phosphine oxide; benzyl-dimethylketal; isopropylthioxanthone; bis(η⁵-2,4-cyclopentadien-1-yl)bis[2,6-difluoro-3-(1H-pyrrol-1-yl)phenyl]titanium); and mixtures of any of the foregoing; wherein all wt% are with respect to the acrylic nail monomer liquid.

In a further embodiment falling within the scope of the claims, the acrylic nail monomer liquid comprises about 50 wt% to about 65 wt% of 2-ethoxyethyl methacrylate; about 17 wt% to about 35 wt% of hydroxypropylmethacrylate; one or more crosslinkers; one or more curing agents; and a pigment; wherein all wt% are with respect to the acrylic nail monomer liquid.

In a further aspect, the invention relates to a method of forming an acrylic nail coating comprising the steps: (a) mixing the acrylic nail monomer liquid comprising about 50 wt% to about 65 wt% of 2-ethoxyethyl methacrylate; about 17 wt% to about 35 wt% of hydroxypropylmethacrylate; one or more crosslinkers; and one or more curing agents; wherein all wt% are with respect to the acrylic nail monomer liquid; with a polymeric powder to create a mixture; (b) placing the mixture onto a nail; and (c) exposing the mixture to a UV light. In this aspect of the invention, the term "about" when referring to a number means ±3%.

In a further aspect, the invention relates to an acrylic nail coating formed by a method of forming an acrylic nail coating comprising the steps: (a) mixing the acrylic nail monomer liquid comprising about 50 wt% to about 65 wt% of 2-ethoxyethyl methacrylate; about 17 wt% to about 35 wt% of hydroxypropylmethacrylate; one or more crosslinkers; and one or more curing agents; wherein all wt% are with respect to the acrylic nail monomer liquid; with a polymeric powder to create a mixture; (b) placing the mixture onto a nail; and (c) exposing the mixture to a UV light. In this aspect of the invention, and the below embodiments, the term "about" when referring to a number means ±3%.

In a further embodiment falling within the scope of the claims, the acrylic nail coating formed by a method of forming an acrylic nail coating comprises the steps: (a) mixing the acrylic nail monomer liquid comprising about 50 wt% to about 65 wt% of 2-ethoxyethyl methacrylate; about 17 wt% to about 35 wt% of hydroxypropylmethacrylate; one or more crosslinkers; and one or more curing agents; wherein all wt% are with respect to the acrylic nail monomer liquid; with a polymeric powder to create a mixture; (b) placing the mixture onto a nail; and (c) exposing the mixture to a UV light; wherein the acrylic nail coating has a Type D Shore Durometer value of greater than 75, as determined by ASTM D2240.

In a further embodiment falling within the scope of the claims, the acrylic nail coating formed by a method of forming an acrylic nail coating comprises the steps: (a) mixing the acrylic nail monomer liquid comprising about 50 wt% to about 65 wt% of 2-ethoxyethyl methacrylate; about 17 wt% to about 35 wt% of hydroxypropylmethacrylate; one or more crosslinkers; and one or more curing agents; wherein all wt% are with respect to the acrylic nail monomer liquid; with a polymeric powder to create a mixture; (b) placing the mixture onto a nail; and (c) exposing the mixture to a UV light, wherein a 0.5 gram sample of the mixture exhibits a temperature increase of less than about 40 °C during the exposure to UV light.

### DETAILED DESCRIPTION OF THE INVENTION

For illustrative purposes, the principles of the present invention are described by referencing various exemplary embodiments thereof. Although certain embodiments of the invention are specifically described herein, one of ordinary skill in the art will readily recognize that the same principles are equally applicable to, and can be employed in other apparatuses and methods. Before explaining the disclosed embodiments of the present invention in detail, it is to be understood that the invention is not limited in its application to the details of any particular embodiment shown. The terminology used herein is for the purpose of description and not of limitation. Further, although certain methods are described with reference to certain steps that are presented herein in certain order, in many instances, these steps may be performed in any order as may be appreciated by one skilled in the art, and the methods are not limited to the particular arrangement of steps disclosed herein.

As used herein and in the appended claims, the singular forms "a", "an", and "the" include plural references unless the context clearly dictates otherwise. The singular form of any class of the ingredients refer not only to one chemical species within that class, but also to a mixture of those chemical species; for example, the term "crosslinker" in the singular form, may refer to a mixture of compounds each of which is also a crosslinker. The terms "a" (or "an"), "one or more" and "at least one" can be used interchangeably herein. It is also to be noted that the terms "comprising", "including", and "having" can be used interchangeably.

The term "about" when referring to a number means ±3%. For example, the phrase "about 50 wt%" refers to a number between and including 48.500 and 51.500. The term "wt%" means percent by weight.

The term "client" refers to a person whose nails are being treated.

The phrase "nail technician" is a worker who typically skilled or licensed in the art of acrylic nail forming. Alternative names for a nail technician may include a manicurist, a cosmetologist, or an operator. Such a person may work for pay at a nail salon, or may be a manicure aficionado.

Under one alternative embodiment of the present invention, the client and the nail technician is the same person. Although the description of the invention below describes the nail technician and the client as two separate individuals, it is understood that the claimed invention and methods are suitable for use by a single person who is both a nail technician and a client.

The phrase "nail coating composition" refers to a lacquer that is suitable to be applied to fingernails or toenails to decorate or protect the nail plates, that when hardens forms a nail coating.

The phrase "nail coating," also sometimes referred to in literature as "nail polish", "nail enamel" or "nail varnish," refers to the hardened, fully cured substance covering a part or all of the nail, and any portions of this substance that extends or is built beyond the free edge of the nail.

The terms "nail", and by extension "fingernail" and "toenail," refer to either a natural nail or artificial nail. The term "nail" also refers to a human nail, as well as to any toughened keratin at the end of a digit of a non-human animal. The phrase "nail polish," also sometimes referred to in literature as "nail enamel" or "nail varnish," is a lacquer that is suitable to be applied to fingernails or toenails to decorate or protect the nail plates.

The term "acrylic" in the phrase "acrylic nail" refers to hardened polymerized composition used in manicure arts, which are composed of any of several types of polyacrylates, or copolymers of various acrylate monomers, or copolymers of various acrylate monomers with any of several non-acrylitic monomers.

When referring to a composition, the definition of the term "acrylate" as referred to in the monomeric form, includes an ester, a salt, or a conjugate base of acrylic acid, with the formula CH₂=C(R)-COO⁻. The definition of the term "acrylate" referred to in the polymeric form includes the repeating unit of an ester, a salt, or a conjugate base of acrylic acid, with the formula -[CH₂-C(R)(COO⁻)]-. Moiety R is a H, or a C₁ to C₄ alkyl group.

The definition of the term "methacrylate" as referred to in the monomeric form includes an ester, a salt, or a conjugate base of methacrylic acid, with the formula CH₂=C(CH₃)-COO⁻. The definition of the term "methacrylate" as referred to in the polymeric form includes an ester, a salt, or a conjugate base of methacrylic acid, with the formula -[CH₂=C(CH₃)-COO⁻]-.

The present invention relates an acrylic nail monomer solution that can be used in a two-part acrylic system. The monomer solution enables a nail technician to form an artificial nail in a way that is similar to traditional two-part systems, but that cures under UV light.

One of the advantages that the present invention provides is a composition and a method of using such composition, which significantly reduces odors associated with a traditional two-part acrylic nail formation.

Another advantage of the present invention is the improvement of cure properties of UV-cured acrylic systems. Such an improvement lies in the combination of improvement of through-cure efficacy, and an increase of surface hardness.

Yet another advantage of the present invention is the reduction of the yellowing of that is problematic with many thermally cured, or UV cured, acrylic systems.

Still another advantage of the present invention compared to a one-part gel system that cures under UV, is the lower cost of the components, and a simpler application procedure.

Yet still another advantage of the present invention compared to the traditional two-part system is the long open time to sculpt the nail. The monomer solution swells the polymer powder and allows for easier application before cure. As the curing polymer swells, the nail extension can be shaped. The curing polymer will firm up, but will still be malleable. The curing polymer won't run once it swells, but it doesn't set until it is cured.

Yet a further advantage of the present invention is the lower exotherm profile compared to similar nail formulations. UV light initiated gel formulas create heat when curing. Such heat can be unpleasant or even painful to the client. This is especially true for if the product is applied in a thick layer, which results in an increase of the heat flux from the gel into the nail bed. It has been observed that the composition of the present invention exhibits a significantly lower exotherm of the polymerization reaction. Because of the lack of such a heat spike, the technician is able to apply the composition to the present invention in thicker layers when creating nail extensions.

A still further advantage of the present invention over some of the odorless systems is that the polymer cures to a hard acrylic nail that is easy to file.

The monomer liquid of the present invention is used in the traditional manner. A brush is wetted with the monomer solution and is then dipped into a polymer powder. The resulting dough comprising a mixture of the monomer solution and polymer powder is then placed on the nail and shaped. The shaped mixture of the present invention is then polymerized using UV light to cure the mixture to give the artificial nail.

The phrase "acrylic nail monomer liquid" or the phrase "monomer liquid" means a liquid used by nail salon technicians to prepare acrylic nails.

The term "liquid" when referring to the monomer liquid means a liquid composition that is sufficiently homogeneous to be used for preparation of acrylic nails. The liquid may be a uniform clear solution, a liquid that displays a Tyndall effect, a colloid, or a suspension in which any fine particles of the suspension do not precipitate during storage time. The liquid may be colorless, or it may be colored.

In one embodiment falling within the scope of the claims, the monomer liquid comprises (a) about 50 wt% to about 65 wt% of 2-ethoxyethyl methacrylate; (b) about 17 wt% to about 35 wt% of hydroxypropylmethacrylate; (c) about 0.15 wt% to about 0.30 wt% of triphenylphosphine; (d) a crosslinker; and optionally (e) a photoinitiator; wherein all %wt are with respect to the acrylic nail monomer liquid and the term "about" when referring to a number means ±3%.

2-Ethoxyethyl methacrylate ("ETMA") is a composition consisting of, or comprising largely of, the compound of formula CH₂=CMe-C(O)-O-(CH₂)₂-O-Et. ETMA, also abbreviated as 2-EOEMA, has both ether and ester groups as compared to most of the vinyl acrylic monomers. It is thought that these oxygen-containing groups not only impart flexibility into the polymer, but also improve its processability and handling and improve compatibility in blends due to hydrogen bonding. ETMA may be obtained from any commercial sources including San Esters Corp. (New York, NY). When referring to a portion of a copolymer, the term "ETMA" or "2-ethoxyethyl methacrylate" refers to the portion of the copolymer originated from the monomer 2-ethoxyethyl methacrylate, and has the structure -[CH₂-C(Me)(COO(CH₂)₂OEt)]-.

The amount of ETMA is about 50 wt% to about 65 wt% with respect to the monomer liquid. Under an alternative embodiment the amount of ETMA is about 55 wt% to about 60 wt%.

Hydroxypropyl methacrylate, or HPMA, is a composition consisting of, or comprising largely of, the compound of formula CH₂=CMe-C(O)-O-C₃H₆OH. HPMA may be obtained from any commercial sources including San Esters Corp. (New York, NY). When referring to a portion of a copolymer, the terms "HPMA" or "hydroxypropyl methacrylate" refers to the portion of the copolymer originated from the monomer HPMA, and has the structure -[CH₂-C(Me)(COO-C₃H₆OH)]-.

Under one embodiment of the present invention, the hydroxypropyl portion of the methacrylate, -C₃H₆OH, has the structure -CH₂-CH₂-CH₂-OH. Under another embodiment the hydroxylpropyl group has the structure -CH₂-CH(OH)-CH₃. Under still another embodiment the hydroxylpropyl has the structure -CH(OH)-CH₂-CH₃. Under yet another embodiment the hydroxylpropyl has the structure -CH(CH₃)-CH₂-OH. Under yet still another embodiment the hydroxylpropyl has the structure -C(OH)(CH₃)-CH₃.

HPMA may contain a mixture of any of the five aforementioned hydroxypropyl methacrylates isomers. For example, HPMA may be a mixture of hydroxypropyl and hydroxyisopropyl methacrylates.

The amount of HPMA is about 17 wt% to about 35 wt% with respect to the monomer liquid. Under an alternative embodiment the amount of HPMA is about 21 wt% to about 30 wt%.

The monomer liquid of the present invention also comprises one or more crosslinkers. A crosslinker is a compound that contains two or more acrylate groups. Crosslinkers are necessary to provide chemical bonding between several monomers, oligomers, polymers or combinations thereof to yield a cross-linked polymeric structure.

Under one embodiment the monomer liquid comprises a single crosslinker. Under another embodiment the monomer liquid comprises two or more different compounds that are crosslinkers.

Examples of crosslinkers include diacrylates, triacrylates, tetraacrylates, pentaacrylates and higher acrylates. Such examples include trimethylolpropane triacrylate, trimethylolethane triacrylate, trimethylolpropane trimethacrylate, trimethylolethane trimethacrylate, tetramethylene glycol dimethacrylate, triethylene glycol dimethacrylate, tetraethylene glycol diacrylate, pentaerythritol diacrylate, penta-erythritol triacrylate, pentaerythritol tetraacrylate, dipentaerythritol diacrylate, dipenta-erythritol triacrylate, dipentaerythritol tetraacrylate, dipentaerythritol pentaacrylate, dipenta-erythritol hexaacrylate, tripentaerythritol octaacrylate, pentaerythritol dimethacrylate, penta-erythritol trimethacrylate, dipentaerythritol dimethacrylate, dipentaerythritol tetramethacrylate, tripentaerythritol octamethacrylate, ethylene glycol diacrylate, 1,3-butanediol diacrylate, 1,3-butanediol dimethacrylate, sorbitol triacrylate, sorbitol tetraacrylate, pentaerythritol-modified triacrylate, sorbitol tetramethacrylate, sorbitol pentaacrylate, sorbitol hexaacrylate, oligoester acrylates and methacrylates, glycerol di- and tri-acrylate, 1,4-cyclohexane diacrylate, bisacrylates and bismethacrylates of polyethylene glycol having a molecular weight of from 200 to 1500, and mixtures thereof.

Under one embodiment the crosslinkers comprise methacrylate groups. Example of such crosslinkers include dimethacrylates, trimethacrylates, tetramethacrylate, and higher methacrylates. Examples of such methacrylic crosslinkers include trimethylolpropane trimethacrylate, trimethylolethane trimethacrylate, tetramethylene glycol dimethacrylate, triethylene glycol dimethacrylate, pentaerythritol dimethacrylate, penta-erythritol trimethacrylate, dipentaerythritol dimethacrylate, dipentaerythritol tetramethacrylate, tripentaerythritol octamethacrylate, 1,3-butanediol dimethacrylate, sorbitol tetramethacrylate, oligoester methacrylates, bismethacrylates of polyethylene glycol having a molecular weight of from 200 to 1500, and mixtures thereof. For example, trimethylolpropane trimethacrylate is a composition consisting of, or comprising largely of, the compound of formula (CH₂=CMe-C(O)-O-CH₂-)₃C-C₂H₅. It is a low volatility trifunctional monomer offering fast cure response in free radical polymerization.

Another suitable crosslinker is an alkoxylated crosslinker, with the formula (CH₂=CMe-C(O)-O-(AO)ₓ-CH₂-)₃C-R; wherein wherein R is a C₁ to C₆ alkyl group; AO is a small alkoxy group, such as an ethylene oxide, -CH₂-CH₂-O-, propylene oxide, -CH(CH₃)-CH₂-O-, -CH₂-CH₂-CH₂-O-, butylene oxide, and -CH(Et)-CH₂-O-; and wherein for each (CH₂=CMe-C(O)-O-(AO)ₓ-CH₂-) group x is independently 0, 1, 2, or 3. Using R = ethyl, and AO = ethylene oxide as an example, the alkoxylated crosslinker would have a structure of formula 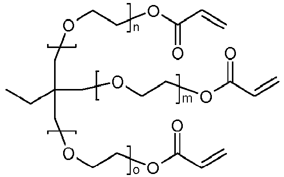 wherein m, n, and o are each independently 0, 1, 2, or 3.

The acrylic nail monomer of the present invention also comprises one or more curing agents. The definition of a curing agent also includes a hardening accelerator.

Examples of a suitable curing agent includes phosphorus-based curing agent, or an amine-based curing agent.

Under one embodiment the monomer liquid comprises a single curing agent. Under another embodiment the monomer liquid comprises two or more different compounds that are curing agents.

Examples of a phosphorus-based curing agent include triphenylphosphine, tributylphosphine, tri(p-methylphenyl)phosphine, tri(nonylphenyl)phosphine, diphenyltolylphosphine, tetraphenylphosphonium bromide, methyltriphenylphosphonium, methyltriphenylphosphponium chloride, methoxymethyltriphenylphosphonium, and benzyltriphenylphosphonium chloride.

Examples of an amine-based curing agent include melamine, imidazole, N,N-dimethyl-p-toluidine (DMPT), N-methyl-N-(2-hydroxyethyl)-p-toluidine (MHPT), and isomeric toluidines.

Under one embodiment the curing agent is a triphenylphosphine. The amount of triphenylphosphine is about 0.15 wt% to about 0.30 wt% with respect to the monomer liquid. Under an alternative embodiment the amount of triphenylphosphine is about 0.20 wt% to about 0.25 wt%.

The monomer liquid of the present invention may also comprise one or more photoinitiators. Such photoinitiators may be selected from benzyl ketones, monomeric hydroxyl ketones, polymeric hydroxyl ketones, α-amino ketones, acyl phosphine oxides, metallocenes, benzophenone, and benzophenone derivatives. Specific examples of photoinitiators include 1-hydroxy-cyclohexylphenylketone; benzophenone; 2-benzyl-2-(dimethylamino)-1-(4-(4-morphorlinyl)phenyl)-1-butanone; 2,2-dimethoxy-2-phenyl acetophenone; 2-methyl-1-(4-methylthio)phenyl-2-(4-morphorlinyl)-1-propanone; 2,4,6-trimethylbenzoyldiphenyl-phosphine oxide; bis(2,4,6-trimethyl benzoyl)phenyl phosphine oxide; diphenyl-(2,4,6-trimethylbenzoyl) phosphine oxide; bis(2,6-dimethoxybenzoyl-2,4,4-trimethyl pentyl)phosphine oxide; 2-hydroxy-2-methyl-1-phenyl-propan-1-one; phenyl bis(2,4,6-trimethylbenzoyl) phosphine oxide; benzyl-dimethylketal; isopropylthioxanthone; bis(η⁵-2,4-cyclopentadien-1-yl)bis[2,6-difluoro-3-(1H-pyrrol-1-yl)phenyl]titanium), and mixtures of any of the foregoing.

The monomer solution of the present application may further comprise additional ingredients, including colorants, dyes, pigments, whiteners, and perfumes.

Dyes and pigments may be included to alter the appearance of the formed artificial nail. Whiteners may be added to the solution to whiten the nail and create a more natural appearance. Examples of whiteners include titanium dioxide. Such whiteners may also used to create special color effects like the white nail tips used in French manicures. Whiteners may also include fluorescent agent to make the nail appear to have albedo greater than 1.

Other colorants may be added to give the nail coating a pinkish color cast. The pinkish case gives a pleasing color to the nail bed.

Alternatively, blue or violet colorants, such as D&C Violet 2, may be added to the monomer solution to counter any yellowing.

Yellowing of the artificial nail may also be mitigated by adding color stabilizers to the monomer liquid. Such compounds absorb ultraviolet light that can cause discoloration of the resins.

According to certain embodiments, the formulations may further comprise at least one UV stabilizing agent. In certain embodiments, the UV stabilizer is present at up to 2 wt%.

The compositions of the invention may contain one or more UV absorbers, which assist in reducing the yellowing which is often seen in artificial nails. UV absorbers have the ability to convert incident UV radiation into less damaging infrared radiation (heat), or visible light. A recommended amount of UV absorber is 0.001-5% by weight of the total composition. Suitable UV absorbers include hydroxy benzotriazole compounds and benzophenone compounds. The acrylic nail monomer liquid may comprise up to 5 wt % of a UV-absorber selected from the group consisting of hydroxy benzotriazole compounds such as 2-(2-hydroxy-5'-methylphenyl)benzotriazole, benzophenones, 1-12,3-benzylidene camphor, benzyl salicylate, borneolone, bumetrizole, PABA, butyl PABA, butyl methoxydibenzoymethane, cinoxate, DEA-methoxycinnamate, dbenzoxazoyl naphthalene, digalloyl trioleate, diisopropyl methyl cinnamate TinuvinP^{®} and mixtures thereof.

Other additives include flow agents to help control how the mixture of the monomer liquid and polymer powder spreads on the surface of the nail.

Any of above monomers, curing agents, adjuncts, and other ingredients as provided by its manufacturer may contain small amounts of additives and impurities. The purity level of the ingredient may be above 90 wt%. Alternatively, the purity level may be above 95 wt%. The purity level may be above 97 wt%. Additives for monomers may include inhibitors such as hydroquinone, HQ, monomethyl ether quinone, MEHQ, and IA. Impurities may include isomers of the monomer, oligomers, unreacted starting materials, water, and solvent used in the formation of the monomers.

Another aspect of the present invention is a method of use of the above-described acrylic nail monomer liquid to form an acrylic nail coating. This method comprises mixing the acrylic nail monomer liquid with a polymeric powder to create a mixture; placing the mixture onto a nail; and exposing the mixture to a UV light.

The polymeric powder is any "polymeric powder" or "polymer" or "powder" that is use in cosmetic nail industry to form acrylic nails. The polymer powder consists of fine microspheres, with a mean diameter between 20 and 100 µm. The polymer powder comprises oligomers, radical intiators, and other components. Examples of a suitable oligomer include poly(ethyl methacrylate), poly(methyl methacrylate), or a co-polymer of methyl methacrylate and ethyl methacrylate. Under one embodiment, polymer may be blended with other polymers to improve its flexibility.

Generally, the monomer liquid of the present invention is used in the traditional manner of forming acrylic nails, followed by an exposure to UV light. A brush is wetted with the monomer solution and is then dipped into a polymer powder. The resulting dough comprising a mixture of the monomer solution and polymer powder is then placed on the nail and shaped. Once the mixture is sculpted into the desired shape, the nail is exposed to UV light.

This method allows for a very long open time to sculpt the nail. The monomer solution swells the polymer powder and allows for easier application before cure. As the curing polymer swells, the nail extension can be shaped. The curing polymer will set but will still be malleable.

After the composition is shaped, the composition, along with finger and the hand of the client, is exposed to UV light to cure the composition. A suitable UV light may be natural sunlight. Another suitable UV light may be a UV lamp, such as a 36 watt lamp commonly used in many nail salons. Such a UV lamp may operate at any wavelength required to cure the photopolymerizable composition, such as between 320 nm and 420 nm. The exposure time should be as long enough to allow for curing of the photopolymerizable composition. This may be 5 seconds to 6 minutes.

The term "UV lamp" is meant to be interpreted broadly. It refers to any source of electromagnetic radiation that exhibits light in the 320 nm to 420 nm range at sufficient enough strength to cure the composition of the present invention. The term "UV lamp" includes traditional UV lamps that contain fluorescent lamps, such as compact fluorescent light bulbs, that give off UV light in the above-described ranges. The term "UV lamp" also refers to newer sources of light or UV radiation, such as light-emitting diode lamps (commonly referred to as "LED lamps") that emit electromagnetic radiation which includes UV light in the 320 nm to 420 nm range at sufficient enough strength to cure the composition of the present invention. The term "UV lamp" also refers to any other type of source of light that comprises UV light in the 320 nm to 420 nm range at sufficient enough strength to cure the composition of the present invention.

The terms "cure", "curing", and like, are used herein are similar to those in the nail art, and are broadly encompassing terms. These term refer to any portion of, or the entire process of polymerization which is experienced under the UV light.

Another aspect of the present invention is an acrylic nail coating formed by a method comprising the steps of mixing the acrylic nail monomer liquid with a polymeric powder to create a mixture; placing the mixture onto a nail; and exposing the mixture to a UV light to form the acrylic nail coating.

Under one embodiment, the acrylic nail coating has improved surface properties over those of the other nail coatings. The hardness may be quantified by the Type D Shore Hardness measurements. This measurement may be evaluated by ASTM D2240, or a similar standard method. Under one embodiment the phrase "ASTM D2240" refers to the latest revision of the standard. Under another embodiment the phrase "ASTM D2240" refers to any of the standards carrying the ASTM D2240 designation, including D2240-15, D2240-05(2010), D2240-05, D2240-04e1, D2240-04, D2240-03, D2240-02b, D2240-02a, D2240-02, D2240-00, and like.

The Type D Shore Durometer value of the nail coating formed by the mixing the acrylic nail monomer liquid with a polymer, placing the mixture onto a nail; and exposing the mixture to a UV light to form the acrylic nail coating, is above 75 durometer units. Under another embodiment the Type D Shore Durometer value is above 80 durometer units.

The present invention is also directed to the acrylic nail monomer liquid which when used with a polymer, exhibits a lower exotherm compared to similar products. The exotherm is the apparent rise in temperature due to the heat generated during the curing reaction caused by UV light. The exotherm is measured on a 0.5 gram sample formed on a glass substrate into a shape approximating a sculpted nail. The exotherm is measured by a thermocouple on the glass substrate to measure the approximate rise in temperatures that a client would feel during the curing process under the UV light. Under one embodiment of the present invention, the exotherm (i.e., the peak temperature achieved during the reaction less the starting temperature) is less than about 40 °C (71 °F). Under another embodiment, the exotherm is less than about 35 °C (63 °F).

The present invention is also directed to the acrylic nail monomer liquid which when used with a polymer resists the yellowing of the nail coating. Such mitigation of yellowing may be observed over time by the use of procedures such as those in ASTM E313.

### Experimental

Eight odor-free monomer liquid compositions were prepared by mixing at least the five following ingredients at various levels: about 50 to about 70 wt% of ETMA; about 10 to about 30 wt% HPMA; about 0.15 to about 0.25 wt% TPP; about 10 to about 20 wt% of one or more crosslinkers; and about 2 to about 4 wt% of one or more photoinitiators. Two of the eight composition also contained up to 8 wt% of additives including one or more antifungal agents, one or more dyes, and one or more plasticizers. The wt% are with respect to the monomer liquid composition. The addition order and preparative methods are similar to those as used the nail composition preparation industry.

The physical and chemical properties of nail coatings prepared by mixing the above-prepared monomer liquid with a polymer, followed by exposing the mixture to a UV light, were compared to the currently available nail formulation. Comparative Examples 1 and 2 (Ex. 1 and Ex. 2) are reduced odor non-curable monomers; working Example 3 (Ex. 3) is selected from one of the eight above-prepared monomer liquid compositions (Ex. 3, working example).

### Hardness Measurements

The comparison of surface properties of nail coating formed by compositions of Examples 1, 2, and 3 were obtained by measuring hardness by Type D Shore Durometer. The method used to gather and evaluate the measurements were similar to those as in ASTM D2240.

**Table 1: Type D Shore Hardness**

| Sample | Ex 1 | Ex 2 | Ex 3 |
|---|---|---|---|
| 1 | 55 | 71 | 73 |
| 2 | 81 | 74 | 79 |
| 3 | 68 | 72 | 80 |
| 4 | 69 | 56 | 82 |
| 5 | 66 | 77 | 82 |
| 6 | 66 | 70 | 81 |
| Average | 68 | 70 | 80 |
| Std Dev | 8 | 7 | 3 |

The above table shows that the working Example 3 has a statistically significant improvement in hardness over commercially available products.

### Exotherm Measurements

Several samples of the each of the coating compositions comprising the compositions were tested for the amount of heat being given off when forming a nail coating. 0.5 g samples were applied to a thermocouple on a glass slide.

**Table 2: Exotherm (°F)**

| Sample | Ex 1 | Ex 2 | Ex 3 |
|---|---|---|---|
| 1 | 153.9 | 147.2 | 129.7 |
| 2 | 165 | 132.6 | 131.2 |
| 3 | 165.2 | 138.4 | 126.5 |
| 4 | 171 | | |
| Average | 163.8 | 139.4 | 129.1 |
| Std Dev | 7.1 | 7.4 | 2.4 |

The above table shows that the working Example 3 has a statistically significant improvement in lowering the exotherm over commercially available products. Whereas the rise in temperature due to the reaction for Ex. 1 was 92 °F (51 °C), and for Ex. 2 was 67 °F (37 °C), the rise for Ex 3. was only 57 °F (32 °C).

### Yellow Index

The Yellow Index ("Yᵢ") for the commercial product Ex. 2 was compared to the Yᵢ of the composition comprising the acrylic nail monomer liquid of several of the working examples.

The samples were prepared and allowed to sit for about three minutes before polymerization, mimicking the time that is typical in a nail salon. The samples were polymerized and the yellow indices were measured to give the "initial" measurements. The Yᵢ was measured on a Konica/Minolta CM3600D Spectrophotometer using Spectra MagicPa NX software. The samples were then left on a laboratory benchtop for 24 h and the yellow indices was measured again.

In a typical comparison, the initial Yellow Index for the commercial product was 15.78, whereas the Yellow Index for the working example was 9.33. Similarly, after the Yellow Index after 24 hours for a commercial product was 9.76, but the Yellow Index for the typical working example was 7.83.

In all cases, working examples exhibit less yellow than a typical system using the commercial UV acrylic monomer liquid. The working examples yield sample that are less yellow immediately after curing and 24 h later as well.

## Claims

1. An acrylic nail monomer liquid comprising:
(a) about 50 wt% to about 65 wt% of 2-ethoxyethyl methacrylate;
(b) about 17 wt% to about 35 wt% of hydroxypropylmethacrylate;
(c) one or more crosslinkers; and
(d) one or more curing agents;
wherein all wt% are with respect to the acrylic nail monomer liquid and the term "about" when referring to a number means ±3%.

2. The acrylic nail monomer liquid of claim 1, comprising about 55 wt% to about 60 wt% of 2-ethoxyethyl methacrylate.

3. The acrylic nail monomer liquid of claim 1 or 2, comprising about 21 wt% to about 30 wt% of hydroxypropylmethacrylate.

4. The acrylic nail monomer liquid of any one of claims 1 to 3, wherein one crosslinker is a polyacrylate selected from the group consisting of a diacrylate, triacrylate, tetraacrylate, pentaacrylate, and a mixture thereof.

5. The acrylic nail monomer of any one of claims 1 to 4, wherein one crosslinker is an alkoxylated crosslinker of formula (CH₂=CMe-C(O)-O-(AO)ₓ-CH₂-)₃C-R;
wherein R is a C₁ to C₆ alkyl group;
AO is an alkoxy group selected from the group consisting of ethylene oxide, -CH₂-CH₂-O-, propylene oxide, -CH(CH₃)-CH₂-O-, -CH₂-CH₂-CH₂-O-, butylene oxide, and
-CH(Et)-CH₂-O-; and
wherein for each CH₂=CMe-C(O)-O-(AO)ₓ-CH₂- group x is independently 0, 1, 2, or 3.

6. The acrylic nail monomer liquid of any one of claims 1 to 5, where one crosslinker is ethoxylated crosslinker of formula (CH₂=CMe-C(O)-O-(CH₂-CH₂-O)*ₓ*-CH₂-)₃C-C₂H₅; wherein for each CH₂=CMe-C(O)-O-(CH₂-CH₂-O)*ₓ*-CH₂- group x is independently 0, 1, 2, or 3.

7. The acrylic nail monomer liquid of any one of claims 1 to 6, wherein the one or more curing agents is selected from the group consisting of triphenylphosphine, tributylphosphine, tri(p-methylphenyl)phosphine, tri(nonylphenyl)phosphine, diphenyltolylphosphine, tetraphenylphosphonium bromide, methyltriphenylphosphonium, methyltriphenylphosphponium chloride, methoxymethyltriphenylphosphonium, benzyltriphenylphosphonium chloride, melamine, imidazole, N,N-dimethyl-p-toluidine (DMPT), N-methyl-N-(2-hydroxyethyl)-p-toluidine (MHPT), isomeric toluidines, and mixtures thereof.

8. The acrylic nail monomer liquid of any one of claims 1 to 7 comprising about 0.15 wt% to about 0.30 wt% of one or more curing agents, wherein the one or more curing agents is triphenylphosphine.

9. The acrylic nail monomer liquid of any one of claims 1 to 8 further comprising a photoinitiator, selected from the group consisting of 1-hydroxy-cyclohexylphenylketone; benzophenone; 2-benzyl-2-(dimethylamino)-1-(4-(4-morphorlinyl)phenyl)-1-butanone; 2,2-dimethoxy-2-phenyl acetophenone; 2-methyl-1-(4-methylthio)phenyl-2-(4-morphorlinyl)-1-propanone; 2,4,6-trimethylbenzoyldiphenyl-phosphine oxide; bis(2,4,6-trimethyl benzoyl)phenyl phosphine oxide; diphenyl-(2,4,6-trimethylbenzoyl) phosphine oxide; bis(2,6-dimethoxybenzoyl-2,4,4-trimethyl pentyl)phosphine oxide; 2-hydroxy-2-methyl-1-phenyl-propan-1-one; phenyl bis(2,4,6-trimethylbenzoyl) phosphine oxide; benzyl-dimethylketal; isopropylthioxanthone; bis(η⁵-2,4-cyclopentadien-1-yl)bis[2,6-difluoro-3-(1H-pyrrol-1-yl)phenyl]titanium); and mixtures of any of the foregoing.

10. The acrylic nail monomer liquid of any one of claims 1 to 9, wherein the acrylic nail monomer liquid further comprises a pigment.

11. A method of forming an acrylic nail coating comprising the steps:
(a) mixing the acrylic nail monomer liquid of any one of claims 1 to 10 with a polymeric powder to create a mixture;
(b) placing the mixture onto a nail; and
(c) exposing the mixture to a UV light.

12. An acrylic nail coating formed by a method comprising the steps:
(a) mixing the acrylic nail monomer liquid of any one of claims 1 to 10 with a polymeric powder to create a mixture;
(b) placing the mixture onto a nail; and
(c) exposing the mixture to a UV light;
to form an acrylic nail coating.

13. The acrylic nail coating of claim 12, wherein the acrylic nail coating has a Type D Shore Durometer value of greater than 75, as determined by ASTM D2240.

14. The acrylic nail coating of claim 12, wherein a 0.5 gram sample of the mixture exhibits a temperature increase of less than about 40 °C during the exposure to UV light.

## Patentansprüche

1. Acrylnagel-Monomerflüssigkeit, umfassend:
(a) etwa 50 Gew.-% bis etwa 65 Gew.-% 2-Ethoxyethylmethacrylat;
(b) etwa 17 Gew.-% bis etwa 35 Gew.-% Hydroxypropylmethacrylat;
(c) einen oder mehrere Vernetzer; und
(d) ein oder mehrere Härtungsmittel;
wobei sich alle Gew.-% auf die Acrylnagel-Monomerflüssigkeit beziehen und der Begriff "etwa" bei Bezugnahme auf eine Zahl ± 3 % bedeutet.

2. Acrylnagel-Monomerflüssigkeit nach Anspruch 1, umfassend etwa 55 Gew.-% bis etwa 60 Gew.-% 2-Ethoxyethylmethacrylat.

3. Acrylnagel-Monomerflüssigkeit nach Anspruch 1 oder 2, umfassend etwa 21 Gew.-% bis etwa 30 Gew.-% Hydroxypropylmethacrylat.

4. Acrylnagel-Monomerflüssigkeit nach einem der Ansprüche 1 bis 3, wobei ein Vernetzer ein Polyacrylat ist, das aus der Gruppe ausgewählt ist, die aus einem Diacrylat, Triacrylat, Tetraacrylat, Pentaacrylat und einer Mischung davon besteht.

5. Acrylnagel-Monomerflüssigkeit nach einem der Ansprüche 1 bis 4, wobei ein Vernetzer ein alkoxylierter Vernetzer der Formel (CH₂=CMe-C(O)-O-(AO)ₓ-CH₂-)₃C-R ist;
wobei R eine C₁ bis C₆ Alkylgruppe ist;
AO eine Alkoxygruppe ist, die aus der Gruppe ausgewählt ist, die aus Ethylenoxid, -CH₂-CH₂-O-, Propylenoxid, -CH(CH₃)-CH₂-O-, -CH₂-CH₂-CH₂-O-, Butylenoxid und -CH(Et)-CH₂-O- besteht; und
wobei für jede CH₂=CMe-C(O)-O-(AO)ₓ-CH₂- Gruppe x unabhängig 0, 1, 2 oder 3 ist.

6. Acrylnagel-Monomerflüssigkeit nach einem der Ansprüche 1 bis 5, wobei ein Vernetzer ein ethoxylierter Vernetzer der Formel (CH₂=CMe-C(O)-O-(CH₂-CH₂-O)ₓ-CH₂-)₃C-C₂H₅ ist; wobei für jede CH₂=CMe-C(O)-O-(CH₂-CH₂-O)ₓ-CH₂- Gruppe x unabhängig 0, 1, 2 oder 3 ist.

7. Acrylnagel-Monomerflüssigkeit nach einem der Ansprüche 1 bis 6, wobei das eine oder die mehreren Härtungsmittel aus der Gruppe ausgewählt sind, die aus Triphenylphosphin, Tributylphosphin, Tri(p-methylphenyl)phosphin, Tri(nonylphenyl)phosphin, Diphenyltolylphosphin, Tetraphenylphosphoniumbromid, Methyltriphenylphosphonium, Methyltriphenylphosphponiumchlorid, Methoxymethyltriphenylphosphonium, Benzyltriphenylphosphoniumchlorid, Melamin, Imidazol, N,N-Dimethyl-p-toluidin (DMPT), N-Methyl-N-(2-hydroxyethyl)-p-toluidin (MHPT), isomeren Toluidinen und Mischungen davon besteht.

8. Acrylnagel-Monomerflüssigkeit nach einem der Ansprüche 1 bis 7, umfassend etwa 0,15 Gew.-% bis etwa 0,30 Gew.-% eines oder mehrerer Härtungsmittel, wobei das eine oder die mehreren Härtungsmittel Triphenylphosphin sind.

9. Acrylnagel-Monomerflüssigkeit nach einem der Ansprüche 1 bis 8, die ferner einen Photoinitiator umfasst, der aus der Gruppe ausgewählt ist, die aus 1-Hydroxycyclohexylphenylketon, Benzophenon; 2-Benzyl-2-(dimethylamino)-1-(4-(4-morphorlinyl)phenyl)-1-butanon; 2,2-Dimethoxy-2-phenylacetophenon; 2-Methyl-1-(4-methylthio)phenyl-2-(4-morphorlinyl)-1-propanon; 2,4,6-Trimethylbenzoyldiphenylphosphinoxid; Bis(2,4,6-trimethylbenzoyl)phenylphosphinoxid; Diphenyl-(2,4,6-trimethylbenzoyl)phosphinoxid; Bis(2,6-dimethoxybenzoyl-2,4,4-trimethylpentyl)phosphinoxid; 2-Hydroxy-2-methyl-1-phenyl-propan-1-on; Phenyl-bis(2,4,6-trimethylbenzoyl)phosphinoxid; Benzyldimethylketal; Isopropylthioxanthon; Bis(η⁵ -2,4-cyclopentadien-1-yl)bis[2,6-difluor-3-(1H-pyrrol-1-yl)phenyl]titan) und Mischungen der vorgenannten Stoffe besteht.

10. Acrylnagel-Monomerflüssigkeit nach einem der Ansprüche 1 bis 9, wobei die Acrylnagel-Monomerflüssigkeit außerdem ein Pigment umfasst.

11. Verfahren zur Herstellung einer Acrylnagelbeschichtung, umfassend die Schritte:
(a) Mischen der Acrylnagel-Monomerflüssigkeit nach einem der Ansprüche 1 bis 10 mit einem polymeren Pulver, um ein Gemisch herzustellen;
(b) Aufbringen des Gemischs auf einen Nagel; und
(c) Aussetzen des Gemischs gegenüber UV-Licht.

12. Acrylnagelbeschichtung, hergestellt durch ein Verfahren, das die folgenden Schritte umfasst:
(a) Mischen der Acrylnagel-Monomerflüssigkeit nach einem der Ansprüche 1 bis 10 mit einem polymeren Pulver, um ein Gemisch herzustellen;
(b) Aufbringen des Gemischs auf einen Nagel; und
(c) Aussetzen des Gemischs gegenüber UV-Licht;
um eine Acrylnagelbeschichtung zu bilden.

13. Acrylnagelbeschichtung nach Anspruch 12, wobei die Acrylnagelbeschichtung einen Shore-Durometer-Wert vom Typ D von mehr als 75 aufweist, bestimmt nach ASTM D2240.

14. Acrylnagelbeschichtung nach Anspruch 12, wobei eine 0,5-Gramm-Probe des Gemischs einen Temperaturanstieg von weniger als etwa 40°C während des Aussetzens gegenüber UV-Licht aufweist.

## Revendications

1. Un liquide monomère d'ongle acrylique comprenant :
(a) environ 50 % en poids à environ 65 % en poids de méthacrylate de 2-éthoxyéthyle ;
(b) environ 17 % en poids à environ 35 % en poids d'hydroxypropyl méthacrylate ;
(c) un ou plusieurs agents de réticulation ; et
(d) un ou plusieurs agents de durcissement ;
dans lequel tous les % en poids sont exprimés par rapport au liquide monomère d'ongle acrylique et le terme « environ », lorsqu'il réfère à un nombre », signifie ± 3 %.

2. Liquide monomère d'ongle acrylique selon la revendication 1, comprenant environ 55 % en poids à environ 60 % en poids de méthacrylate de 2-éthoxyéthyle.

3. Liquide monomère d'ongle acrylique selon la revendication 1 ou 2, comprenant environ 21 % en poids à environ 30 % en poids d'hydroxypropyl méthacrylate.

4. Liquide monomère d'ongle acrylique selon l'une quelconque des revendications 1 à 3, dans lequel un agent de réticulation est un polyacrylate choisi dans le groupe constitué par un diacrylate, un triacrylate, un tétraacrylate, un pentaacrylate et un mélange de ceux-ci.

5. Monomère d'ongle acrylique selon l'une quelconque des revendications 1 à 4, dans lequel un agent de réticulation est un agent de réticulation alcoxylé de formule (CH₂=CMe-C(O)-O-(AO)ₓ-CH₂-)₃C-R ;
dans laquelle R est un groupe alkyle en C₁ à C₆ ;
AO est un groupe alcoxy choisi dans le groupe constitué par l'oxyde d'éthylène,-CH2-CH2-O-, l'oxyde de propylène, -CH(CH₃)-CH₂-O-, -CH₂-CH₂-CH₂-O-, l'oxyde de butylène, et
-CH(Et)-CH₂-O- ; et
dans lequel pour chaque groupe CH₂=CMe-C(O)-O-(AO)ₓ-CH₂-, x est indépendamment 0, 1, 2 ou 3.

6. Liquide monomère d'ongle acrylique selon l'une quelconque des revendications 1 à 5, dans lequel un agent de réticulation est un agent de réticulation éthoxylé de formule (CH₂=CMe-C(O)-O-(CH₂-CH₂-O)ₓ-CH₂-)₃C-C₂H₅ ;
dans lequel pour chaque CH₂=CMe-C(O)-O-(CH₂-CH₂-O)ₓ-CH₂-, x est indépendamment 0, 1, 2 ou 3.

7. Liquide monomère d'ongle acrylique selon l'une quelconque des revendications 1 à 6, dans lequel le ou les agents de durcissement sont choisis dans le groupe constitué par la triphénylphosphine, la tributylphosphine, la tri(p-méthylphényl)phosphine, la tri(nonylphényl)phosphine, la diphényltolylphosphine, le bromure de tétraphénylphosphonium, le eméthyltriphénylphosphonium, le chlorure de méthyltriphénylphosphponium, le méthoxyméthyltriphénylphosphonium, le chlorure de benzyltriphénylphosphonium, la mélamine, l'imidazole, la N,N-diméthyl-p-toluidine (DMPT), la N-méthyl-N-(2-hydroxyéthyl)-p-toluidine (MHPT), les toluidines isomères et leurs mélanges.

8. Liquide monomère d'ongle acrylique selon l'une quelconque des revendications 1 à 7, comprenant environ 0,15 % en poids à environ 0,30 % en poids d'un ou plusieurs agents de durcissement, dans lequel le ou les agents de durcissement sont la triphénylphosphine.

9. Liquide monomère d'ongle acrylique selon l'une quelconque des revendications 1 à 8, comprenant en outre un photo-initiateur, choisi dans le groupe constitué par la 1-hydroxy-cyclohexylphénylcétone ; la benzophénone ; la 2-benzyl-2-(diméthylamino)-1-(4-(4-morphorlinyl)phényl)-1-butanone ; la 2,2-diméthoxy-2-phényl acétophénone ; la 2-méthyl-1-(4-méthylthio)phényl-2-(4-morphorlinyl)-1-propanone ; l'oxyde de 2,4,6-triméthylbenzoyldiphényl-phosphine ; l'oxyde de bis (2,4,6-triméthylbenzoyl)phényl-phosphine ; l'oxyde de diphényl-(2,4,6-triméthylbenzoyl)-phosphine ; l'oxyde de bis(2,6-diméthoxybenzoyl-2,4,4-triméthylpentyl)-phosphine ; la 2-hydroxy-2-méthyl-1-phényl-propan-1-one ; l'oxyde de phényl bis(2,4,6-triméthylbenzoyl)-phosphine ; le benzyl-diméthylcétal ; l'isopropylthioxanthone ; le bis(η⁵-2,4-cyclopentadién-1-yl)bis[2,6-difluoro-3-(1H-pyrrol-1-yl)phényl] titane) ; et des mélanges de l'un quelconque des précédents.

10. Liquide monomère d'ongle acrylique selon l'une quelconque des revendications 1 à 9, dans lequel le liquide monomère d'ongle acrylique comprend en outre un pigment.

11. Procédé de formation d'un revêtement d'ongle acrylique comprenant les étapes consistant à :
(a) mélanger le liquide monomère d'ongle acrylique selon l'une quelconque des revendications 1 à 10 avec une poudre polymère pour créer un mélange ;
(b) placer le mélange sur un ongle ; et
(c) exposer le mélange à une lumière UV.

12. Revêtement d'ongle acrylique formé par un procédé comprenant les étapes consistant à :
(a) mélanger le liquide monomère d'ongle acrylique selon l'une quelconque des revendications 1 à 10 avec une poudre polymère pour créer un mélange ;
(b) placer le mélange sur un ongle ; et
(c) exposer le mélange à une lumière UV ;
pour former un revêtement d'ongle acrylique.

13. Revêtement d'ongle acrylique selon la revendication 12, dans lequel le revêtement d'ongle acrylique a une valeur au duromètre Shore de type D supérieure à 75, telle que déterminée par ASTM D2240.

14. Revêtement d'ongle acrylique selon la revendication 12, dans lequel un échantillon de 0,5 gramme du mélange présente une augmentation de température inférieure à environ 40°C lors de l'exposition à la lumière UV.
